Europäisches Patentamt

**European Patent Office**

**Office européen des brevets**

(11) Numéro de publication: **0 107 575**
A1

# (12) DEMANDE DE BREVET EUROPEEN

(21) Numéro de dépôt: **83401990.3**

(22) Date de dépôt: **12.10.83**

(51) Int. Cl.³: **A 61 M 37/00**

(30) Priorité: **12.10.82 FR 8217074**

(43) Date de publication de la demande: **02.05.84**
**Bulletin 84/18**

(84) Etats contractants désignés: **AT BE CH DE FR GB IT LI LU NL SE**

(71) Demandeur: **LABORATOIRES FOURNIER S.A. Société anonyme dite:, 9 rue Petitot, F-21100 Dijon (FR)**

(72) Inventeur: **Mikler, Claude, 117 Avenue du Drapeau, F-21100 Dijon (FR)**
Inventeur: **Perego, Jean, 59, rue Orfila, F-75020 Paris (FR)**

(74) Mandataire: **Cliscl, Serge, CABINET BEAU DE LOMENIE 55 rue d'Amsterdam, F-75008 Paris (FR)**

(54) **Dispositif pour l'administration percutanée de principe actif.**

(57) La présente invention a trait à un dispositif pour l'administration percutanée de principe actif. Ce dispositif, qui comprend un moyen pour mettre en équilibre la concentration, au voisinage de la peau, d'un principe actif à administrer contenu dans une préparation médicamenteuse, avec la concentration dudit principe actif dans la peau, est constitué par une chambre dite de percutanéation auverte sur la peau, une chambre de stockage, et, une cloison séparant les deuc chambres qui est munie d'au moins une fenêtre pour communication entre les deux chambres, l'alimentation de la chambre de percutanéation étant réalisée en continu.

Dispositif pour l'administration percutanée de principe actif.

La présente invention a trait à un nouveau dispositif pour l'administration de principes actifs à travers la peau.

Dans ce qui suit par principe actif, on entend toute substance thérapeutique active et efficace ; par médicament, ledit principe actif ou tout mélange d'au moins deux principes actifs, et, par préparation médicamenteuse, toute composition renfermant au moins un principe actif en association avec un véhicule ou excipient physiologiquement acceptable.

On sait que pour résoudre le problème technique de l'administration percutanée de médicaments un certain nombre de solutions a été préconisé. On connaît notamment du brevet français n°6733 M, un dispositif comprenant un support, un matériau à structure foliée renfermant un anesthésique local, et, le cas échéant un adhésif ; de la demande de brevet français publiée n°2 437 830, un dispositif comprenant un support et un polymère microporeux renfermant un médicament (dans le cas d'espèce de la nitroglycérine disposée dans les lacunes de la structure microporeuse du polymère) ; de la demande de brevet français publiée n°2 479 089, un dispositif comprenant un support et une masse adhésive qui renferme de la nitroglycérine ; du brevet américain n°3 996 934 un pansement comprenant un support, une matrice renfermant des microcapsules et une membrane pour déterminer la vitesse de libération du médicament contenu dans les microcapsules ; et de la demande de brevet français publiée n°2 397 190, un dispositif comprenant un support imperméable, un gel renfermant des particules d'un médicament, une membrane microporeuse, une couche adhésive perméable au médicament devant venir au contact de la peau, pouvant contenir une certaine quantité de celui-ci, et revêtue d'une couche protectrice enlevable avant mise en place sur la peau.

On sait également, notamment, des brevets français n° 1 549 286, n° 967 763 et n° 1 126 230 que l'on a envisagé des dispositifs comprenant une chambre de percutanéation ouverte sur la peau du type ventouse alimentée en discontinu par un réservoir de stockage. Il se trouve ces dispositifs, qui aspirent la peau par dépression ou qui simultanément aspirent la peau par dépression et la repoussent par compression, ne sont guère d'un usage pratique et n'ont jamais pu être utilisés de façon efficace.

On connaît par ailleurs, un autre dispositif qui a été envisagé, notamment dans le brevet américain n° 3 053 255, qui comprend (i) un support fibreux absorbant contenant un véhicule physiologiquement acceptable, (ii) un support fibreux absorbant contenant le principe actif à administrer et destiné à venir en contact avec la peau, et, (iii) un moyen étanche séparant les deux supports absorbants et muni d'un organe, en particulier une mèche, pour mise en contact desdits supports lors de l'utilisation. L'inconvénient d'un tel dispositif est de limiter le flux transcutanée au moment de la mise en place puis lors de l'utilisation après ladite mise en place.

Il se trouve que les solutions antérieurement connues ne sont pas pratiques dès lors qu'elles limitent le flux transcutané, la concentration du principe actif au contact de l'épiderme et celle du même principe actif dans la peau n'étant pas en équilibre à chaque instant.

Le principal barrage à la percutanéation d'un principe actif est l'épiderme. En effet, l'épiderme, qui peut être schématisé selon une succession de couches plus ou moins perméables, a un comportement différent vis-à-vis des diverses molécules qui peuvent être à son contact. Le flux transcutané pourra être plus ou moins rapide en fonction de la molécule à délivrer et en fonction de sa formulation. Mais l'épiderme va toujours se comporter comme un réservoir; pour que le flux transcutané soit constant, il est important que les concentrations du principe actif (i) au contact de la peau, et (ii) dans la peau, soient sensiblement constantes et identiques pendant toute la durée d'application.

Il est également nécessaire d'apporter, rapidement, au moment de la pose du dispositif, la quantité suffisante de principe actif pour imprégner l'épiderme de façon à ce que le flux transcutané s'établisse rapidement.

Selon l'invention on préconise une nouvelle solution technique qui satisfait aux exigences de la percutanéation d'un principe actif.

Le dispositif selon l'invention pour l'administration percutanée de principe actif comprenant un réservoir de stockage et une chambre dite de percutanéation ouverte sur la peau, est caractérisé en ce qu'il comprend :

(i) une première chambre contenant une préparation médicamenteuse renfermant un principe actif à administrer, ladite chambre faisant office de réservoir de stockage,

(ii) une deuxième chambre pour l'administration percutanée du principe actif faisant office de chambre de percutanéation, et

(iii) une cloison séparant lesdites chambres et munie d'au moins une fenêtre formant diaphragme et assurant l'alimentation en continu de la chambre de percutanéation en préparation médicamenteuse provenant de la chambre de stockage,

l'ensemble de la chambre de stockage, de la chambre de percutanéation et de la cloison les séparant, qui est munie d'au moins une fenêtre, constituant un moyen mettant, après application sur la peau, en équilibre la concentration au voisinage de la peau du principe actif à administrer contenu dans la préparation médicamenteuse, avec la concentration dudit principe actif dans la peau.

Le dispositif selon l'invention comprend de façon avantageuse une chambre de stockage d'une préparation médicamenteuse munie d'une face rentrante qui est pourvue d'au moins une fenêtre formant diaphragme pour l'alimentation en continu de la chambre de percutanéation, et en ce que ladite chambre de percutanéation est formée par l'espace compris entre ledit diaphragme et la peau quand ledit dispositif d'administration est mis en place sur la peau par l'intermédiaire d'un moyen de liaison étanche.

Selon un mode préféré de réalisation, la chambre de stockage se présentera sous la forme d'une cuvette pourvue du côté de son ouverture d'une cloison qui assure la fermeture dudit réservoir, constitue la face rentrante sus-visée et est munie d'au moins une fenêtre pour mettre en communication l'intérieur de la chambre de stockage avec la chambre de percutanéation constituée par l'espace ménagé entre ladite cloison formant diaphragme et la peau.

Selon ce mode préféré on a en bref un dispositif du type comprenant deux cuvettes emboîtées de façon étanche l'une dans l'autre, le fond de la cuvette pénétrant dans l'autre cuvette étant muni d'au moins une fenêtre, la chambre de stockage étant alors constituée par l'espace compris entre les fonds de chaque cuvette. L'une au moins de ces cuvettes présentera de façon avantageuse un prolongement annulaire du côte de son ouverture. C'est ce prolongement annulaire qui, revêtu d'une couche adhésive sur au moins une partie de sa face externe devant venir en contact avec la peau, assurera une liaison étanche avec celle-ci. La liaison étanche entre les deux cuvettes sera réalisée soit par contact de la surface latérale externe de la cuvette rentrante avec la surface latérale interne de l'autre cuvette, soit par contact desdites surfaces latérales, d'une part, et d'au moins une partie des prolongements annulaires de chaque cuvette, d'autre part, soit encore par contact desdits prolongements annulaires. Les deux cuvettes peuvent être emboîtées à force, collées au moyen d'un adhésif et/ou encliquetées.

La chambre de stockage (de même que le dispositif d'administration qui la comprend) doit être imperméable vis à vis de la préparation médicamenteuse. Elle peut être, par exemple, en métal, en carton paraffiné, ou de préférence en plastique. Elle peut avoir une forme quelconque mais de préférence on fera appel à une forme facile à réaliser industriellement, notamment une forme cylindrique, tronconique ou sphérique.

Outre son rôle de stockage de la préparation médicamenteuse, la chambre de stockage doit fournir, au moment de la mise en service, la quantité requise de ladite préparation pour remplir la chambre de percutanéation. Pour ce faire, on pourra, lors du remplissage de la chambre réservoir, mettre la préparation en surpression de façon à ce qu'elle s'écoule dans la chambre de percutanéation après ouverture du diaphragme. Un moyen préféré consiste à prévoir qu'une partie du réservoir soit déformable, sa partie supérieure de préférence, la déformation appliquée à la mise en service correspondant exactement au volume de la chambre de percutanéation. Le réservoir peut, le cas échéant, être cloisonné de façon à contenir notamment différentes préparations médicamenteuses qui seront mélangées extemporanément dans la chambre de percutanéation au moment de la mise en service par autant de fenêtres que de compartiments réalisés par cloisonnement.

L'organe de fermeture du réservoir, à savoir la cloison formant diaphragme, c'est-à-dire la cuvette qui est munie d'au moins une fenêtre et qui pénètre dans l'autre cuvette peut être réalisée en un matériau identique à celui du réservoir ou différent. La ou les fenêtres prévues sur ladite cloison présentent chacune une très faible surface utile par rapport à la surface de la cloison en regard de la préparation médicamenteuse dans la chambre de percutanéation au moment de l'emploi, le rapport de la surface utile de chaque fenêtre à celle de la cloison étant avantageusement compris entre $10^{-5}$ et $10^{-1}$.

Chaque fenêtre est, pour le conditionnement du dispositif selon l'invention, équipée d'un moyen d'obturation enlevable au moment de la mise en place dudit dispositif sur la peau. Ce moyen d'obturation assure la fermeture du réservoir après son remplissage.

Le médicament stocké dans le réservoir se présente sous la forme d'une préparation médicamenteuse renfermant, en association avec un véhicule physiologiquement acceptable, au moins un principe actif. Le véhicule peut être notamment soit un solvant tel que l'éthanol ou un mélange de solvants, soit encore un gel. Le flux médicamenteux, à travers l'épiderme, pourra être contrôlé par addition en quantité plus ou moins importante d'un solvant qui accélèrera ou diminuera la vitesse de percutanéation, comme par exemple l'eau, le DMSO, le DMF, l'éther, l'acétone ou le propylèneglycol. La vitesse de percutanéation pourra encore être contrôlée par gélification plus ou moins importante de la préparation médicamenteuse.

Avec le dispositif selon l'invention on pourra administrer par voie percutanée un certain nombre de principes actifs, notamment des vaso-dilatateurs (de préférence la trinitrine et ses analogues ou ses dérivés) des vaso-constricteurs , des hypotenseurs (tels que la clonidine et ses dérivés), des anti-tussifs, des bronchodilatateurs, des hormones naturelles ou synthétiques (par exemple, la testostérone ou l'un de ses dérivés), des antalgiques, des anti- inflammatoires, des anti-asthmatiques, des relaxants musculaires.

Pour fournir au moment de la mise en place sur la peau, la quantité de principe actif nécessaire pour imprégner l'épiderme, on a intérêt à utiliser une préparation médicamenteuse la plus concentrée possible, et à faire appel à un véhicule ne passant sensiblement pas la barrière cutanée, à la différence de l'enseignement du brevet américain n° 3 053 255 précité. On pourra, par exemple, utiliser une préparation médicamenteuse sous forme de solution saturée, du principe actif solide pouvant alors être présent dans le réservoir ; dans ce cas le principe actif solide passe en solution dans le réservoir au fur et à mesure que le principe actif solubilisé, présent dans la chambre de percutanéation, pénètre dans l'épiderme.

De façon avantageuse, en vue d'assurer rapidement l'établissement du flux transcutané, on préconise que le rapport du volume de la chambre de percutanéation à celui de la chambre de stockage soit inférieur ou égal à 0,1 et de préférence compris entre 0,08 et 0,005.

D'autres avantages et caractéristiques de l'invention seront mieux compris à la lecture qui va suivre des dessins annexés, donnés à tire d'illustration et nullement limitatifs, où

- la figure 1 est une représentation en coupe d'un dispositif pour administration percutanée selon l'invention ;

- la figure 2 est une représentation en coupe d'un dispositif analogue à celui de la figure 1 et montrant quelques variantes de réalisation ;

- la figure 3 est une vue en perspective d'un dispositif selon l'invention, et

- les figures 4 et 5 sont des représentations partielles en coupe de dispositifs selon l'invention montrant des variantes de réalisation en ce qui concerne la cloison du réservoir de stockage.

Le dispositif selon l'invention comprend une chambre de percutanéation 1 et une chambre constituant un réservoir de stockage 2 d'une préparation médicamenteuse. La chambre de percutanéation 1 qui est constituée par l'espace compris entre la peau 3 et la cloison 4 du réservoir de stockage, est en communication avec ledit réservoir par l'intermédiaire d'au moins une fenêtre 5 prévue dans ladite cloison 4. La fenêtre 5 permet l'alimentation en continu de la chambre 1 en préparation médicamenteuse contenue dans le réservoir 2. Ce dispositif se présente sous la forme de deux cuvettes emboîtées, la cuvette extérieure 6 et la cuvette intérieure constituée par la cloison 4 logée dans la cuvette extérieure étant en contact étanche par leurs parois latérales et / ou leurs prolongements annulaires 7 et 8.

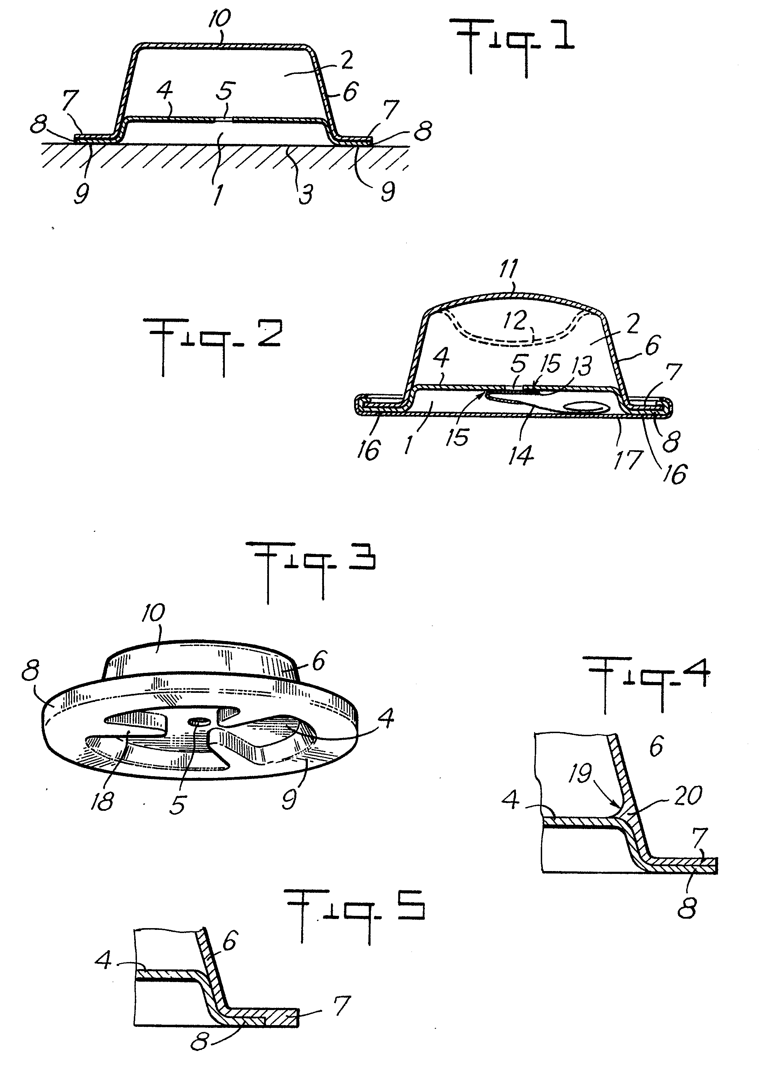

Le dispositif étant mis en place, la peau 3 est en contact étanche avec au moins un desdits prolongements annulaires sur au moins une portion de la surface externe dudit prolongement. Selon la figure 1 la liaison étanche entre le prolongement annulaire 8 et la peau 3 est réalisée sur la face 9 dudit prolongement annulaire au moyen d'une couche adhésive non représentée ici. Par ailleurs, la partie supérieure du réservoir ou fond 10 de la cuvette externe est susceptible d'être déformable.

La figure 2 montre le fond de la cuvette externe avant déformation en 11, et le même fond après déformation en 12. La figure 2 représente également une variante du dispositif selon l'invention avant mise en place sur la peau. La fenêtre 5 est munie d'un opercule d'obturation 13 enlevable au moment de l'emploi. Cet opercule est lié en 15 à la cloison 4 par une couche adhésive (non représentée) au voisinage de la fenêtre 5. L'opercule 13 est solidaire d'une languette 14 qui permet son enlèvement lors de la mise en place du dispositif sur la peau. Par ailleurs, selon la réalisation de la figure 2, il est prévu que le prolongement annulaire 7 s'encliquète dans le rebord du prolongement annulaire 8, et que, le cas échéant, un moyen de protection temporaire 17 de la chambre 1, notamment pour conserver stérile son intérieur avant

utilisation , est lié en 16 au prolongement annulaire 8. Lors de la mise en place du dispositif sur la peau, on enlève la protection 17 (qui est notamment soit une feuille de papier siliconé, soit une feuille d'aluminium) et avantageusement on colle la face 9 sur la peau de façon que la languette 14 soit comprise entre la face 9 et la masse adhésive, cette disposition permet en tirant sur cette languette d'ouvrir la fenêtre 5 et d'éliminer l'air contenu dans la chambre de percutanéation, par l'intermédiaire d'un petit canal dans la masse adhésive.

Le cas échéant, on peut avoir intérêt à prévoir dans la chambre de percutanéation un moyen de rigidification maintenant constant le volume de ladite chambre en conservant les distances séparant la cloison 4 de la peau. Ce moyen peut être constitué par des bossages représentés en 18 sur la figure 3 ou des entretoises.

La figure 5 montre un dispositif selon l'invention dans lequel le prolongement annulaire 8 est logé dans une platine annulaire ménagée dans le prolongement 7.

La figure 4 montre un dispositif dans lequel la paroi de la cuvette 6 présente au voisinage de la cloison rentrante 4 une surface concave 19 et un accroissement de matière 20 pour assurer un contact étanche entre la cuvette externe et la cuvette interne, d'une part, et guider la préparation médicamenteuse contenue dans le réservoir vers la cloison 4, d'autre part.

Pour permettre un passage efficace de la préparation médicamenteuse du réservoir de stockage à la chambre de percutanéation, on peut, le cas échéant, concevoir une fenêtre 5 tronconique dont le diamètre en regard de la chambre de stockage est plus grand que celui qui est en regard de la chambre de percutanéation.

10

Le dispositif selon l'invention a permis d'administrer avec succès par voie percutanée, soit de la testostérone à des sujets souffrant d'insuffisance androgénique, soit de la trinitrine à des sujets souffrant d'angine de poitrine. On a ainsi administré 3 mg de testotérone par jour et pendant 8 jours à d'aide du dispositif selon l'invention dans lequel la chambre de percutanéation a un diamètre moyen de 21 mm et une profondeur moyenne de 0,3 mm, la fenêtre a un diamètre moyen de 2,6 mm et la chambre de stockage a une hauteur moyenne de 6 mm pour un diamètre moyen de 20,5 mm, de façon à contenir 75 mg de testostérone en solution dans de l'éthanol.

Le dispositif selon l'invention est utile en thérapeutique humaine et vétérinaire pour l'administration percutanée, notamment en remplacement des implants.

11

## R E V E N D I C A T I O N S

1.      Dispositif pour l'administration percutanée de principe actif, du type comprenant un réservoir de stockage et une chambre dite de percutanéation ouverte sur la peau, caractérisé en ce qu'il comprend :

(i) une première chambre contenant une préparation médicamenteuse renfermant un principe actif à administrer, ladite chambre faisant office de réservoir de stockage,

(ii) une deuxième chambre pour l'administration percutanée du principe actif faisant office de chambre de percutanéation, et

(iii) une cloison séparant lesdites chambres et munie d'au moins une fenêtre formant diaphragme et assurant l'alimentation en continu de la chambre de percutanéation en préparation médicamenteuse provenant de la chambre de stockage,

l'ensemble de la chambre de stockage, de la chambre de percutanéation et de la cloison les séparant qui est munie d'au moins une fenêtre constituant un moyen mettant, après application sur la peau, en équilibre la concentration au voisinage de la peau du principe actif à administrer contenu dans la préparation médicamenteuse, avec la concentration dudit principe actif dans la peau.

2.      Dispositif selon la revendication 1, caractérisé en ce que la chambre de stockage est munie d'une face rentrante pourvue d'au moins une fenêtre formant diaphragme pour l'alimentation en continu de la chambre de percutanéation, et en ce que ladite chambre de percutanéation est formée par l'espace compris entre ledit diaphragme et la peau lorsque ledit dispositif d'administration est mis en place sur la peau par l'intermédiaire d'un moyen de liaison étanche.

3.      Dispositif selon la revendication 1, caractérisé en ce que la chambre de stockage se présente sous la forme d'une cuvette pourvue du côté de son ouverture d' une cloison pénétrante dans ladite cuvette, assurant la séparation de la chambre de stockage avec la chambre de percutanéation, et munie d'au moins une fenêtre mettant en communication lesdites chambres.

4. Dispositif selon la revendication 1, caractérisé en ce qu'il comprend deux cuvettes emboîtées l'une dans l'autre de façon étanche, le fond de la cuvette interne logée dans la cuvette externe étant muni d'au moins une fenêtre, la chambre de stockage étant constituée par l'espace compris entre les fonds de chaque cuvette, et la chambre de percutanéation étant constituée par l'espace compris entre le fond de la cuvette interne formant diaphragme et la peau,lorsque ledit dispositif est mis en place sur la peau.

5. Dispositif selon la revendication 4, caractérisé en ce que au moins l'une des deux cuvettes est pourvue du côté de son ouverture d'un prolongement annulaire qui, revêtu d'une couche adhésive sur au moins une partie de sa face externe devant venir en contact avec la peau, assure une liaison étanche avec celle-ci.

6. Dispositif selon la revendication 1, caractérisé en ce que la chambre de stockage comprend au moins deux compartiments isolés, chaque compartiment ne communiquant qu'avec la chambre de percutanéation.

7. Dispositif selon la revendication 1, caractérisé en ce que la préparation médicamenteuse contenue dans la chambre de stockage renferme le principe actif en association avec un solvant organique en tant que véhicule pharmaceutiquement acceptable.

8. Dispositif selon la revendication 1, caractérisé en ce que la préparation médicamenteuse contenue dans la chambre de stockage renferme le principe actif en association avec un gel en tant que véhicule pharmaceutiquement acceptable.

9. Dispositif selon la revendication 1, caractéri-

0107575

13

sé en ce que le rapport du volume de la chambre de percutanéation à celui de la chambre de stockage est inférieur ou égal à 0,1 et de préférence compris entre 0,08 et 0,005.

10. Dispositif selon la revendication 1, caractérisé en ce que le rapport de la surface utile de chaque fenêtre à celle de la cloison séparant la chambre de stockage de la chambre de percutanéation est compris entre $10^{-5}$ et $10^{-1}$.

11. Dispositif selon la revendication 1, caractérisé en ce que la chambre de stockage est déformable de façon à·remplir la chambre de percutanéation au moment de la mise en place sur la peau.

12. Dispositif selon la revendication 1, caractérisé en ce que chaque fenêtre est munie d'un moyen d'obturation enlevable au moment de la mise en place sur la peau.

13. Dispositif selon la revendication 1 caractérisé en ce que la chambre de percutanéation comprend un moyen de rigidification disposé entre la cloison formant diaphragme et la peau.

14. Dispositif selon la revendication 13, caractérisé en ce que le moyen de rigidification est choisi parmi l'ensemble constitué par les entretoises et les bossages.

0107575

1/1

Fig. 1

Fig. 2

Fig. 3

Fig. 4

Fig. 5

Office européen
des brevets

## RAPPORT DE RECHERCHE EUROPEENNE

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| X | BE-A- 677 945 (RECKITT & COLEMAN) <br> * Page 3, lignes 14-16; page 6, lignes 18-32; page 8, lignes 7-18; figures 4,9 * | 1,3,4, 11 | A 61 M  37/00 |
| Y | | 13,14 | |
| | --- | | |
| Y,D | FR-A-1 126 230 (J. LEDOCTE et al.) <br> * Page 3, colonne 1, lignes 24-28; figures * | 13,14 | |
| | --- | | |
| X,D | US-A-3 053 255 (F. MEYER) <br><br> * Colonne 3, ligne 14 - colonne 4, ligne 63; colonne 5, lignes 9-36; colonne 6, lignes 53-65; revendication 4; figures 3,4 * | 1-5,7-12 | |
| | --- | | |
| X | FR-A-2 143 564 (ALZA CORPORATION) <br> * Page 5, lignes 18-27; page 7, ligne 36 - page 8, ligne 5; page 9, lignes 1-5; page 13, lignes 10-13, 25-27; page 16, lignes 3-12 * | 1,2,6-8 | DOMAINES TECHNIQUES RECHERCHES (Int. Cl. ³) <br><br> A 61 M <br> A 61 B |
| | --- | | |
| X,D | FR-A- 967 763 (G. STAMPE) <br> * Page 3, lignes 1-9; figure 7 * | 1,2,12 | |
| | ---     -/- | | |

Le présent rapport de recherche a été établi pour toutes les revendications

| Lieu de la recherche <br> LA HAYE | Date d'achèvement de la recherche <br> 11-01-1984 | Examinateur <br> WOLF C.H.S. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

 

& : membre de la même famille, document correspondant

OEB Form 1503 03 82

**Office européen des brevets**

# RAPPORT DE RECHERCHE EUROPEENNE

| DOCUMENTS CONSIDERES COMME PERTINENTS | | | Page 2 |
|---|---|---|---|
| Catégorie | Citation du document avec indication, en cas de besoin, des parties pertinentes | Revendication concernée | CLASSEMENT DE LA DEMANDE (Int. Cl. 3) |
| X | BE-A- 827 706 (J. SILVER)<br><br>* Page 3, lignes 15-29; page 4, lignes 20-22; figure 2 * | 1,3,4, 11 | |
| A | | 2 | |
| | --- | | |
| A | GB-A-1 510 569 (AMERICAN HOME PRODUCTS)<br>* Page 1, lignes 39-50, 63-89 * | 1,2,5-8,11 | |
| | --- | | |
| A | US-A-2 561 071 (H.C. PRISK)<br><br>* Colonne 1, lignes 29-41; colonne 2, lignes 29-44; figures * | 2,5,7, 11 | |
| | --- | | DOMAINES TECHNIQUES RECHERCHES (Int. Cl. 3) |
| A | FR-A-2 292 489 (FISONS LTD.)<br>* Page 2, lignes 4-14; revendication 6 * | 4-8 | |
| | ----- | | |

Le présent rapport de recherche a été établi pour toutes les revendications

| Lieu de la recherche<br>LA HAYE | Date d'achèvement de la recherche<br>11-01-1984 | Examinateur<br>WOLF C.H.S. |
|---|---|---|